# EUROPEAN PATENT SPECIFICATION

(11) **EP 4 453 017 B1**
(45) Date of publication and mention of the grant of the patent: **16.09.2026**
(21) Application number: 22844067.3
(22) Date of filing: 22.12.2022
(51) Int. Cl.: C07K 14/605

(54) **PROCESSES FOR SYNTHESIZING GLUCAGON-LIKE-PEPTIDE 2 (GLP-2) ANALOGUES**
VERFAHREN ZUR SYNTHESE VON GLUCAGON-LIKE-PEPTID-2(GLP-2)-ANALOGA
PROCÉDÉS DE SYNTHÈSE D'ANALOGUES DU PEPTIDE 2 DE TYPE GLUCAGON (GLP-2)

(30) Priority: 23.12.2021 EP 21217600
(43) Date of publication of application: 30.10.2024
(73) Proprietor: Zealand Pharma A/S, 2860 Søborg (DK)
(72) Inventor: MELANDER, Claes, 2860 Søborg (DK); MALIK, Leila, 2860 Søborg (DK); PAWLAS, Jan, 200 61 Limhamn (SE); HANSEN, Stefan, 200 61 Limhamn (SE)
(74) Representative: Mewburn Ellis LLP
(86) International application number: PCT/EP2022/087440
(87) International publication number: WO 2023/118416

(56) References cited:
- WO-A1-2018/229252
- "Amino Acids, Peptides and Proteins in Organic Chemistry : Building Blocks, Catalysis and Coupling Chemistry vol. 3 Building Blocks, Catalysis and Coupling Chemistry", vol. 3, 15 December 2010, WILEY-VCH VERLAG GMBH & CO. KGAA, Weinheim, Germany, ISBN: 978-3-527-32102-5, article F ALBERICIO ET AL: "10 Fmoc Methodology: Cleavage from the Resin and Final Deprotection", pages: 349 - 369, XP055554734, DOI: 10.1002/9783527631803.ch10
- J-C M MONBALIU ET AL: "Recent trends in Cys- and Ser/Thr-based synthetic strategies for the elaboration of peptide constructs", CHEMICAL COMMUNICATIONS, vol. 48, no. 95, 12 September 2012 (2012-09-12), pages 11601 - 11622, XP055571155, DOI: 10.1039/c2cc34434c

## Description

### Field of the Invention

The present invention relates to processes for obtaining glucagon-like-peptide-2 (GLP-2) analogues.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH (SEQ ID NO: 10), where Hy is hydrogen. It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not as useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day.

WO 2006/117565 describes GLP-2 analogues which comprise one of more substitutions as compared to [hGly²]GLP-2 and which improved biological activity *in vivo* and/or improved chemical stability, e.g. as assessed in *in vitro* stability assays. In particular, GLP-2 analogues are described which have substitutions at one or more of positions 8, 16, 24 and/or 28 of the wild-type GLP-2 sequence, optionally in combination with further substitutions at position 2 and one or more of positions 3, 5, 7, 10 and 11, and/or a deletion of one or more of amino acids 31 to 33. These substitutions may also be combined with the addition of a N-terminal or C-terminal stabilizing peptide sequence.

Among the molecules disclosed in WO 2006/117565 is ZP1848 (also referred to as glepaglutide) which has been designed to have improved chemical stability and/or biological activity. Dosage regimes for GLP-2 analogues including ZP1848 and its metabolites (that is, glepaglutide) are described in WO 2018/229252.

### Summary of the Invention

Broadly, the present invention is directed to improved methods for the synthesis and purification of glucagon-like-peptide-2 (GLP-2) analogues such as ZP1848. The invention is as defined in the appended claims.

Accordingly, the present invention relates to methods for producing a glucagon-like peptide 2 (GLP-2) analogue synthesized by solid phase peptide synthesis (SPPS), wherein the GLP-2 analogue is represented by the formula:
R¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R² (SEQ ID NO: 11)
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z² is absent or a peptide sequence of 1-6 amino acid units of Lys;
or a pharmaceutically acceptable salt or derivative thereof.

In a first aspect, the invention provides a method of producing a GLP-2 analogue synthesized by solid phase peptide synthesis (SPPS) comprising the steps of:
i) loading a C8 or C18 column with the crude GLP-2 analogue;
ii) adjusting the pH of the column with a first buffer system, wherein the first buffer system is a phosphate buffer system;
wherein step ii) includes sequential use of said buffer system to raise the pH of the column to greater than 7.5 while the GLP-2 analogue is on the column followed by lowering the pH of the column to an acidic pH.

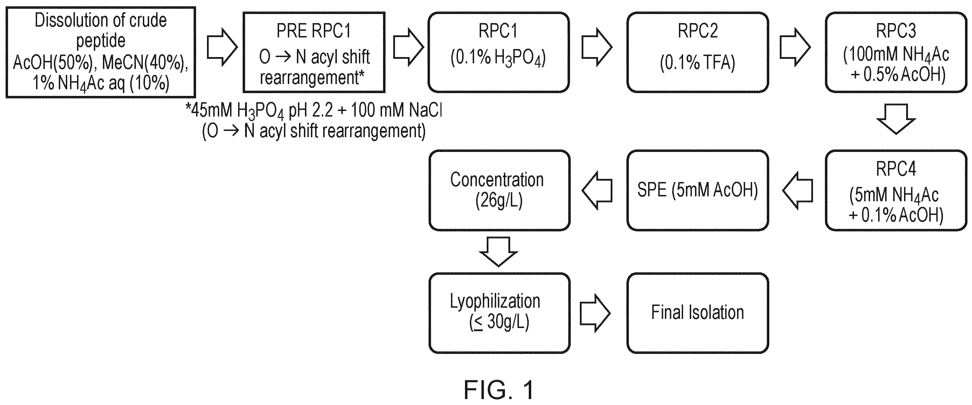

In the present context, and as shown in Figure 1, the step of adjusting the pH on the column is called "Pre-RPC1". During this Pre-RPC1, an O → N acyl shift occurs. It will be appreciated that the acyl shift occurs while the peptide is on the column. In other words, step ii) includes passing the isoelectric point of the peptide while the peptide is on the column to perform an O → N acyl shift.

In more detail, during peptide cleavage of the GLP-2 analogue in published methods, an N → O acyl shift takes place. Such acyl shifts are recognized in the art, and it is known that adjusting the pH of the peptide in solution to neutral and back to acidic conditions prior to subsequent purification steps reduced these impurities, in effect performing an O → N acyl shift.

Prior to the present invention, this pH adjustment was performed in solution. However, for the GLP-2 analogues of the present invention, undesirable precipitation is observed. Surprisingly, the inventors have found that this O → N acyl shift can be performed on a column, avoiding loss of material and processing complications arising from the precipitation.

This increase in pH of the peptide on the column constitutes, to the inventors' knowledge, a new general methodology and in effect performs this 'conversion' or 'purification' step on the column rather than in solution.

In other words, the invention provides a method of performing an O → N acyl shift on a GLP-2 analogue while the GLP-2 analogue is on the column (Pre-RPC1).

The use of phosphate buffer in peptide syntheses of this type is desirable because it is known to be efficient to remove unwanted oligomers and C-terminal deamidation products. However, GLP-2 analogues of the claimed structure were known to precipitate out in a phosphate buffer, making the use of phosphate buffer problematic for the O → N acyl shift. It is therefore even more surprising that the inventors are able to use a phosphate buffer avoiding the loss of material. This performance of the acyl shift on the column using the pH adjustment step provides a further advantage by saving a process step (a separate pH adjustment in solution).

The first buffer system is a phosphate buffer system. In other words, the first buffer system is based on phosphate buffers / phosphoric acid.

The isoelectric point pH(I) of a peptide may be calculated using methods known in the art or may be measured experimentally. It is the pH at which the overall charge of a molecule is zero (neutral charge) or is electrically neutral in the statistical mean.

It will be appreciated that, in the present invention, the pH of the column is increased from an acidic pH. That is, increasing the pH while the peptide is on the column comprises raising the pH of the column to greater than 7.5.

Accordingly, in the present invention the process for purifying a GLP-2 analogue synthesized by solid phase peptide synthesis (SPPS) comprises the steps of:
i) loading a C8 or C18 column with the crude GLP-2 analogue;
ii) adjusting the pH of the column with a first buffer system, wherein the first buffer system is a phosphate buffer system;
wherein step ii) includes sequential use of buffers to raise the pH of the column to greater than 7.5 then to lower the pH of the column to acidic pH. **[*Pre-RPC1*]**

An acidic pH is a pH less than 7, for example less than 5, for example less than 3. Suitably, the pH of the column is lowered to ≤2.5, that is 2.5 or less.

Suitably, step ii) effects an O → N acyl shift on the column and hydrolyses acetylated and trifluoro-acetylated impurities. Doing this on the column prevents an undesired precipitation while achieving the desired chemical transformations.

In examples described herein, the column is a C18 column. In some embodiments, the column is a C18 column. In some embodiments, the column is a C8 column.

Advantageously, this O → N acyl shift on the column can be achieved on the same column as the subsequent purification step.

The present invention further provides a method of purifying a GLP-2 analogue synthesized by SPPS through a four-step chromatographic purification process (referred to herein as RPC1-4). As described above, an O → N acyl shift on the column (Pre-RPC1) can be performed on the same column as the first 'purification' step (RPC1). Alternatively, the four-step chromatographic purification process may be performed on a GLP-2 analogue synthesized by SPPS which has already undergone a pH adjustment process in solution to perform the O → N acyl shift.

In some embodiments, the present invention may provide a five-step chromatographic purification process for purifying a GLP-2 analogue synthesized by SPPS, the method comprising:
*(1) **Pre-RPC1***
   i) loading a C8 or C18 column with the GLP-2 analogue;
   ii) adjusting the pH of the column with a first buffer system, wherein the first buffer system is a phosphate buffer system;
   wherein step ii) includes sequential use of buffers to raise the pH of the column to greater than 7.5 then to lower the pH of the column to acidic pH; then
*(2) **RPC1***
   i) eluting a pool containing the GLP-2 analogue using the first buffer system; then
*(3) **RPC2***
   i) loading a column with the pool containing the GLP-2 analogue obtained in step (2); and
   ii) washing the column with a second buffer system to elute a pool containing the GLP-2 analogue;
   wherein the second buffer system includes trifluoroacetic acid; then
*(4) **RPC3***
   i) loading a column with the pool containing the GLP-2 analogue obtained in step (3); and
   ii) washing the column with a third buffer system to elute a pool containing the GLP-2 analogue;
   wherein the third buffer system includes acetic acid/ammonium acetate; then
*(5) **RPC4***
   i) loading a column with the pool containing the GLP-2 analogue obtained in step (4); and
   ii) washing the column with a fourth buffer system to elute a pool containing the GLP-2 analogue;
   wherein the fourth buffer system includes acetic acid/ammonium acetate.

It will be appreciated that the pool containing the GLP-2 analogue obtained from any step may be loaded directly onto a column for the next step or may be concentrated by evaporation.

In some embodiments, the method further comprises a desalting step in which the GLP-2 analogue obtained in step (5) is loaded onto a column and washed with a buffer system comprising 10 mM AcOH and acetonitrile.

In further embodiments, the invention further comprises the method of synthesising a GLP-2 analogue by solid phase peptide synthesis (SPPS), wherein Z² is a peptide sequence of 1-6 amino acid units of Lys and wherein at least one of the Lys units in Z² is protected with a trityl protecting group during the synthesis.

The trityl group is:

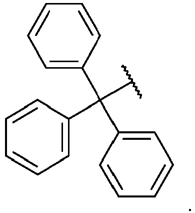

Suitably, Z² is a peptide sequence of 2-6 amino acid units of Lys and the method comprises the steps of:
i) attaching a first P-Lys(Trt)-OH to a solid-state peptide resin with a linker;
ii) removing the P group from the Lys(Trt) amino acid unit;
iii) attaching a second P-Lys(Trt)-OH to the Lys(Trt) amino acid unit attached to the solid-state peptide resin with a linker;
iv) removing the P group from the second Lys(Trt) amino acid unit;
v) attaching subsequent amino acid units until the GLP-2 analogue is synthesized;
vi) cleaving the GLP-2 analogue from the solid-state peptide resin; and
vii) purifying the GLP-2 analogue.
where each P is a protecting group, for example Fmoc.

Employing this Lys(Trt) stratagem during the SPPS results in eradication of all the Lys(Boc) → Lys(t-Bu) add on t-Bu peaks present in the Lys(Boc) crude product, including the most problematic one present at relative retention time 1.1.

In some embodiments, Z² is a peptide sequence of 6 amino acid units of Lys and Lys³⁹ and Lys³⁸ are attached as P-Lys(Trt)-OH. In some embodiments, the remaining Lys amino acid units of Z² are attached as P-Lys(Boc)-OH. That is, the lysine tail during synthesis is [K(Boc)]₄[K₍Trt)]₂.

Surprisingly, the inventors found that trityl protection of 2 out of the 6 amino acid units of Lys, for example the protection of Lys³⁹ and Lys³⁸, was enough to prevent the formation of the *t*-Bu impurities from the crude product. Without wishing to be bound by theory, the rationale for using Lys(Trt) for Lys³⁹ and Lys³⁸ is because: i) using Lys(Boc) in Fmoc/*t*-Bu SPPS caused formation of Lys-t-Bu adducts (Pawlas et al., Peptides, 2014, 108); and ii) unpublished research by PolyPeptide Group found that Lys(Boc) side chains which are closest to the C-terminus (i.e. Lys³⁹ and Lys³⁸ in the peptides described herein) are responsible for the formation of add-on *t*-Bu impurities eluting closest to the main peak. The inventors have observed that Lys(Boc) residues further away from the C-terminus cause the formation of further eluting +56 Da adducts and are in that sense less problematic during downstream processing. Based on these reasons, it is therefore reasonable to use Lys(Trt) solely for those Lys positions understood to pertain to the formation of critical Lys-t-Bu adducts, that is Lys³⁸ & Lys³⁹.

Embodiments of the present invention will now be described by way of example and not limitation with reference to the accompanying figures. However, various further aspects and embodiments of the present invention will be apparent to those skilled in the art in view of the present disclosure.
"and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Brief Description of the Figures

**Figure 1** shows an overview of the O → N acyl shift (Pre-RPC1) and the four-step (RPC1-4) chromatographic purification process for purifying a GLP-2 analogue synthesized by SPPS.
**Figure 2** shows graphs of three syntheses of ZP1848 by +56 Da EIC MS. Main peak at ca. 28 min. Top panel: purified ZP1848 from a synthesis with [K(Boc)]₆ - note the remaining +56 Da impurity. Middle panel: crude from a synthesis with [K(Boc)]₆. Bottom panel: crude from a synthesis with [K(Trt)]₆.
**Figure 3** shows graphs of two Fmoc-K₆-NH₂ crudes by +56 Da EIC MS. Top panel: crude from a [K(Boc)]₆ resin. Bottom panel: crude from a [K(Boc)]₄[K(Trt)]₂ resin.
**Figure 4** shows graphs after the cleavage of ZP1848. Top graph: After 1½ hours in cleavage solution (procedure as **Example 1.11).** Bottom graph: After pH treatment in RPC1 (procedure as **Example 1.12).**

### Detailed Description of the Invention

The present invention provides methods for the purification of a GLP-2 analogue. As described herein, the purification steps are performed on a C8 or C18 column.

Chromatographic methods of purification are well-known in the art. In one aspect, the present invention is directed to the surprising insight that a purification step involving chemical modification (namely, an acyl shift) can be performed on the column rather than in solution. The invention further provides a four-step chromatographic purification process for GLP-2 analogues. As described herein, this four-step chromatographic purification process produces the GLP-2 in high yield with low levels of impurities.

Accordingly, the processes of the invention for purifying the GLP-2 analogue suitably comprise a series of column-based processes. These processes comprise a step of loading a column with a solution containing the GLP-2 analogue then washing and eluting with a buffer system, in each case to obtain a pool comprising the GLP-2 analogue. Each washing process comprises more than one step. It will be appreciated that, as is normal in chromatographic purification, changing the eluent (buffer) is typically achieved by applying a gradient.

In some embodiments, the process for purifying a GLP-2 analogue synthesized by solid phase peptide synthesis (SPPS) comprises the steps of:
i) loading a C8 or C18 column with the GLP-2 analogue;
ii) adjusting the pH of the column by washing with a first buffer system;

wherein the first buffer system comprises:
   Buffer A: 0.1% H₃PO₄;
   Buffer B: MeCN;
   Buffer C: 45mM H₃PO₄ pH 2.2 + 100mM NaCl; and
   Buffer D: 45mM H₃PO₄ pH 7.5-8.0 + 100mM NaCl;
wherein the column is pre-equilibrated with a mixture of 95% Buffer C and 5% Buffer B;
and wherein washing is in the following sequence:
   Step 1: a mixture of 95% Buffer C and 5% Buffer B;
   Step 2: a mixture of 95% Buffer D and 5% Buffer B until the pH is greater than 7.5;
   Step 3: a mixture of 95% Buffer C and 5% Buffer B until the pH is less than 2.5

Step 1 equalises or equilibrates the column, as is conventional in column-based purification.

Steps 2 and 3 may be referred to herein as "Pre-RPC1". As described herein, advantageously the pH adjustment in Pre-RPC1 effects the O → N acyl shift conventionally performed in solution to remove impurities arising from an unwanted N → O acyl shift during the synthesis.

After Step 3 the column may be washed by a solution containing 93% A and 7% B. Suitably, the washing further comprises:
Step 4: linearly increasing the content of Buffer B from 7% to 22% (Buffer A from 93% to 78%), suitably over 12 column volumes; and
Step 5: a mixture of 22% Buffer B and 78% Buffer A, suitably until elution is complete, to obtain a pool contain the GLP-2 analogue.

Steps 4 and 5 are elution of the GLP-2 analogue and is referred to herein as "RPC1" or the "first dimension" chromatographic purification of the peptide. This purification is a consequence of the difference in interaction between the peptide and the impurities with the column media and elution media.

In some embodiments, the process for purifying a GLP-2 analogue synthesized by solid phase peptide synthesis (SPPS) further comprises the steps of:
iii) loading a column with the pool containing the GLP-2 analogue obtained in step ii); and
iv) washing the column with a second buffer system;

wherein the second buffer system 2 comprises:
   Buffer A: 0.1% TFA; and
   Buffer B: MeCN;
wherein the column is pre-equilibrated with a mixture of 90% Buffer A and 10% Buffer B;
and wherein washing is in the following sequence:
   Step 1: a mixture of 90% Buffer A and 10% Buffer B;
   Step 2: linearly increasing the content of Buffer B from 10% to 19% (Buffer A from 90% to 81%), suitably over 1 column volume; and
   Step 3: linearly increasing the content of buffer B from 19% to 30% (Buffer A from 81% to 70%), suitably over 12 column volumes, then continuing washing the column to elute a pool containing the GLP-2 analogue.

Steps iii) and iv) may be referred to herein as "RPC2" or "second dimension".

In some embodiments, the process for purifying a GLP-2 analogue synthesized by solid phase peptide synthesis (SPPS) further comprises the steps of:
v) loading a column with the pool containing the GLP-2 analogue obtained in step iv); and
vi) washing the column with a third buffer system;

wherein the column is pre-equilibrated with a mixture of 85% Buffer A and 15% Buffer B
wherein the third buffer system comprises:
   Buffer A: 100 mM NH₄OAc + 0.5% AcOH; and
   Buffer B: MeCN;
and wherein washing is in the following sequence:
   Step 1: a mixture of 85% Buffer A and 15% Buffer B;
   Step 2: linearly increasing the content of Buffer B from 15% to 29% (buffer A from 85% to 71%), suitably over 1 column volume;
   Step 3: linearly increasing the content of Buffer B from 29% to 37% (buffer A from 71% to 63%), suitably over 10 column volumes; and
   Step 4: a mixture of 30% Buffer A and 70% Buffer B to elute a pool containing GLP-2 analogue.

Steps v) and vi) may be referred to herein as "RPC3" or "third dimension".

In some embodiments, the process for purifying a GLP-2 analogue synthesized by solid phase peptide synthesis (SPPS) further comprises the steps of:
vii) loading a column with the pool containing the GLP-2 analogue obtained in step vi); and
viii) washing the column with a fourth buffer system;

wherein the fourth buffer system comprises:
   Buffer A: 5 mM NH₄OAc + 0.1% AcOH;
   Buffer B: MeCN; and
   Buffer C: 100 mM NH₄OAc + 0.5% AcOH;
wherein the column is pre-equilibrated with a mixture of 90% Buffer C and 10% Buffer B;
and wherein washing is in the following sequence:
   Step 1: a mixture of 90% Buffer C and 10% Buffer B, suitably 1 column volume;
   Step 2: a mixture of 90% Buffer A and 10% Buffer B, suitably 1 column volume;
   Step 3: linearly increasing the content of Buffer B from 10% to 13% (buffer A from 90% to 87%), suitably over 1 column volume; and
   Step 4: linearly increasing the content of Buffer B from 13% to 25% (buffer A from 87% to 75%), suitably over 12 column volumes, to elute a pool containing the GLP-2 analogue.

Steps vii) and viii) may be referred to herein as "RPC4" or "fourth dimension".

In some embodiments, the process for purifying a GLP-2 analogue synthesized by solid phase peptide synthesis (SPPS) further comprises the steps of:
ix) loading a column with the pool containing the GLP-2 analogue obtained in step viii); and
x) washing the column with a desalting buffer system;

wherein the desalting buffer system comprises:
   Buffer A: 10 mM AcOH; and
   Buffer B: MeCN
wherein the column is pre-equilibrated with a mixture of 95% A and 5% B;
and wherein the washing is in the following sequence:
   Step 1: a mixture of 95% A and 5% B; and
   Step 2: linearly increasing the content of buffer B from 5% to 50% (buffer A from 95% to 50%), suitably over 1 column volume.

Steps ix) and x) may be referred to as "desalting" or "SPE".

The process for purifying a GLP-2 analogue synthesized by solid phase peptide synthesis (SPPS) may further comprise a lyophilization step.

In further embodiments, the invention further comprises the method of synthesising a GLP-2 analogue by solid phase peptide synthesis (SPPS), wherein Z² is a peptide sequence of 1-6 amino acid units of Lys and wherein at least one of the Lys units in Z² is protected with a trityl protecting group during the synthesis.

In some embodiments where Z² is a peptide sequence of 1-6 amino acid units of Lys, the method of synthesizing the GLP-2 analogue by solid phase peptide synthesis (SPPS) comprises the steps of:
i) attaching Fmoc-Lys(Trt)-OH to a solid-state peptide resin with a linker;
ii) removing the Fmoc group from the Lys(Trt) amino acid unit;
iii) attaching subsequent amino acid units until the GLP-2 analogue is synthesized;
iv) cleaving the GLP-2 analogue from the solid-state peptide resin; and
v) purifying the GLP-2 analogue.

In some embodiments where Z² is a peptide sequence of 2-6 amino acid units of Lys, the method of synthesizing the GLP-2 analogue comprises the steps of:
i) attaching a first Fmoc-Lys(Trt)-OH to a solid-state peptide resin with a linker;
ii) removing the Fmoc group from the Lys(Trt) amino acid unit;
iii) attaching a second Fmoc-Lys(Trt)-OH to the Lys(Trt) amino acid unit attached to the solid-state peptide resin with a linker;
iv) removing the Fmoc group from the second Lys(Trt) amino acid unit;
v) attaching subsequent amino acid units until the GLP-2 analogue is synthesized;
vi) cleaving the GLP-2 analogue from the solid-state peptide resin; and
vii) purifying the GLP-2 analogue.

Where Z² is a peptide sequence of 6 amino acid units of Lys, the motif may therefore be [K(Boc)]₄[K(Trt)]₂. In other embodiments, the motif may be [K(Boc)]₄[K(Trt)]₂, [K(Boc)]₃[K(Trt)]₃, [K(Boc)]₂[K(Trt)]₄, [K(Boc)]₁[K(Trt)]₅, or [K(Trt)]₆. That is, step subsequent Lys amino acid units attached in (v) may be protected with Trt or Boc.

In some embodiments, the motif is [K(Boc)]₄[K(Trt)]₂ or [K(Trt)]₆. In some embodiments, the motif is [K(Boc)]₄[K(Trt)]₂

### Definitions

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration.

### GLP-2 Analogues

The glucagon-like peptide 2 (GLP-2) analogues of the present invention are represented by the formula:
R¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH;
   and
Z² is absent or a peptide sequence of 1-6 amino acid units of Lys;
or a pharmaceutically acceptable salt or derivative thereof.

Z² is present and/or absent or a peptide sequence of 1-6 amino acid units of Lys, i.e., 1, 2, 3, 4, 5 or 6 Lys residues. The Lys residues may have either D- or L-configuration, but preferably have an L-configuration. Particularly preferred sequences Z² are sequences of four, five or six consecutive lysine residues, and particularly six consecutive lysine residues. Exemplary sequences Z are shown in WO 01/04156.

In some embodiments, R¹ is hydrogen. In some embodiments, X5 is Thr. In some embodiments, X11 is Ala. In some embodiments, R² is NH₂.

In some embodiments, Z² is a peptide sequence of 1-6 amino acid units of Lys. In some embodiments, Z² is a peptide sequence of 2-6 amino acid units of Lys. In some embodiments, Z² is a peptide sequence of 3-6 amino acid units of Lys. In some embodiments, Z² is a peptide sequence of 4-6 amino acid units of Lys. In some embodiments, Z² is a peptide sequence of 5-6 amino acid units of Lys. In some embodiments, Z² is a peptide sequence of 6 amino acid units of Lys. In some embodiments, Z² is a peptide sequence of 1-2 amino acid units of Lys.

In some embodiments of the present invention, in the above formula, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); or
ZP2530 H-HGEGTFSSELATILDALAARDFIAWLIATKITD-OH (SEQ ID NO: 6).

In some embodiments of the present invention, the glucagon-like peptide 2 (GLP-2) analogue is
ZP1848 H-HGEGTFSSELATILDALAARDFIAWLIATKITDKKKKKK-NH₂ (SEQ ID NO: 1).

That is, the GLP-2 analogue is represented by the formula:
H-His¹-Gly²-Glu³-Gly⁴-Thr⁵-Phe⁶-Ser⁷-Ser⁸-Glu⁹-Leu¹⁰-Ala¹¹-Thr¹²-Ile¹³-Leu¹⁴-Asp¹⁵-Ala¹⁶-Leu¹⁷-Ala¹⁸-Ala¹⁹-Arg²⁰-Asp²¹-Phe²²-Ole²³-Ala²⁴-Trp²⁵-Leu²⁶-lle²⁷-Ala²⁸-Thr²⁹-Lys³⁰-Ile³¹-Thr³²-Asp³³-Lys³⁴-Lys³⁵-Lys³⁶-Lys³⁷-Lys³⁸-Lys³⁹-NH₂.

In some embodiments of the present invention, in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

In an embodiment of the present invention, the glucagon-like peptide 2 (GLP-2) analogue is ZP1846 H-HGEGSFSSELSTILDALAARDFIAWLIATKITDKKKKKK-NH₂(SEQ ID NO: 7).

That is, the GLP-2 analogue is represented by the formula:
H-His¹-Gly²-Glu³-Gly⁴-Ser⁵-Phe⁶-Ser⁷-Ser⁸-Glu⁹-Leu¹⁰-Ser¹¹-Thr¹²-Ile¹³-Leu¹⁴-Asp¹⁵-Ala¹⁶-Leu¹⁷-Ala¹⁸-Ala¹⁹-Arg²⁰-Asp²¹-Phe²²-Ile²³-Ala²⁴-Trp²⁵-Leu²⁶-Ile²⁷-Ala²⁸-Thr²⁹-Lys³⁰-Ile³¹-Thr³²-Asp³³-Lys³⁴- Lys³⁵- Lys³⁶-Lys³⁷-Lys³⁸-Lys³⁹-NH₂.

In some embodiments, the GLP-analogue is selected from:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1846 H-HGEGSFSSELSTILDALAARDFIAWLIATKITDKKKKKK-NH₂₍SEQ ID NO: 7);
ZP1855 H-HGEGSFSSELSTILDALAARDFIAWLIATKITD-NH₂ (SEQ ID NO: 8); or
ZP2242 H-HGEGSFSSELSTILDALAARDFIAWLIATKITDK-OH (SEQ ID NO: 9).

The present invention is directed to methods of making and purifying GLP2 analogues. These peptides are intended for use as drug substances. The invention includes GLP analogues obtained by the methods of the invention.

It should be understood that the peptides (drug substance) of the invention might be provided in the form of a salt or other derivative. Accordingly, it will be understood that, after purification and optional further steps, the peptide may be finally obtained as a salt or other derivative. Salts include pharmaceutically acceptable salts, such as acid addition salts and basic salts. Examples of acid addition salts include hydrochloride salts, citrate salts, chloride salts and acetate salts. Preferably, the salt is acetate. In general, it is preferred that the salt is not a chloride salt. Examples of basic salts include salts where the cation is selected from alkali metals, such as sodium and potassium, alkaline earth metals, such as calcium, and ammonium ions ⁺N (R³) ₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences", 17th edition. Ed. Alfonso R. Gennaro (Ed.), Mark Publishing Company, Easton, PA, U.S.A., 1985 and more recent editions, and in the Encyclopaedia of Pharmaceutical Technology.

Preferably, the salt is acetate, which may be obtained following steps RPC1-RPC4 and subsequent desalting as described herein.

In preferred embodiments, the acetate salt of a GLP-2 analogue of the invention is selected from the group consisting of ZP1848-acetate, ZP2949-acetate, ZP2711-acetate, ZP2469-acetate, ZP1857-acetate, ZP2530-acetate, ZP1846-acetate, ZP1855-acetate and ZP2242-acetate. In the present context, the term "ZP1848-acetate" refers to the ZP1848 molecule in the form of an acetate salt. The acetate salts of GLP-2 analogues may be represented by the formula (GLP-2 analogue), x(CH₃COOH) where x is 1.0 to 8.0, i.e. where x is 1.0, 2.0, 3.0, 4.0, 5.0, 6.0, 7.0 or 8.0. In any composition of the acetate salts of the GLP-2 analogues, there may be molecules with different number of acetate molecules so that x is not necessarily a whole integer. In some cases, x is from 4.0 to 8.0, x is from 6.0 to 8.0, or x is from 4.0 to 6.5. In some cases, x is from 4.0 to 6.0, x is from 2.0 to 7.0, x is from 3.0 to 6.0, x is from 4.0 to 6.0, or x is 4.0 to 8.0. Further discussion of acetate salts of GLP-2 analogues as defined in the invention may be found in WO2020/265064.

In a preferred embodiment, the GLP-2 analogue is finally obtained as ZP1848-acetate or H-HGEGTFSSELATILDALAARDFIAWLIATKITDKKKKKK-NH₂ acetate (SEQ ID NO: 1) or (H-HGEGTFSSELATILDALAARDFIAWLIATKITDKKKKKK-NH₂), x(CH₃COOH) where x is 1.0 to 8.0.

A solid composition comprising an acetate salt of a glucagon-like peptide 2 (GLP-2) analogue may be obtained, for example by lyophilization. The solid compositions are useful for formulating with the excipients used to make liquid formulations. For example, a solid composition comprising an acetate salt of a glucagon-like peptide 2 (GLP-2) analogue having the formula:
(H-HGEGTFSSELATILDALAARDFIAWLIATKITDKKKKKK-NH2), x(CH₃COOH) where x is 1.0 to 8.0
may be obtained.

An upper limit of 8.0 acetate molecules per GLP-2 analogue equates to an acetate content of less than 11% acetate and may be formulated to have a viscosity between 0.8 and 2.0 mPa/sec measured at 25°C.

The range of the number of acetate molecules associated with each molecule of the GLP-2 analogues defines a molecular weight range for this component of the formulation. For example, for the acetate salts of ZP1848, the range of the number of acetate molecules associated with each molecule of the GLP-2 analogues defines a molecular weight range of the ZP1848-acetate. By way of example, 1 acetate equivalent with each molecule of ZP1848 provides a molecular weight = 4316 + 60 = 4376 Da. Accordingly, the molecular weights for increasing acetate equivalents with ZP1848 are as follows: 1 acetate equivalent = 4376 Da; 2 acetate equivalents = 4436 Da; 3 acetate equivalents = 4496 Da; 4 acetate equivalents =4556 Da; 5 acetate equivalents = 4616 Da; 6 acetate equivalents = 4676 Da; 7 acetate equivalents = 4736 Da and 8 acetate equivalents = 4796 Da. This in turn defines molecular weight ranges as follows: 1-8 acetate equivalents = 4376 Da - 4796 Da; 4-8 acetate equivalents = 4556 Da - 4796 Da and 6-8 acetate equivalents = 4676 Da - 4796 Da. Further discussion of acetate salts of GLP-2 analogues as defined in the invention may be found in WO2020/265064.

Other derivatives of the GLP-2 analogues of the invention include coordination complexes with metal ions such as Mn²⁺ and Zn²⁺, esters such as *in vivo* hydrolysable esters, free acids or bases, hydrates, or lipids. Esters can be formed between hydroxyl or carboxylic acid groups present in the compound and an appropriate carboxylic acid or alcohol reaction partner, using techniques well known in the art.

### Medical Conditions

The GLP-2 analogue formulations of the present invention are useful as a pharmaceutical agent as described in, for example, WO2020/065064 at page 26 ("Medical Conditions").

### EXAMPLES

### Materials and Methods

Standard equipment and raw materials for good manufacturing practice (GMP) manufacturing of therapeutic peptides known in the art are used throughout.

### EXAMPLE 1: General peptide synthesis

### 1.1 Attachment of Ramage-linker and first step of downloading via sub-stoichiometric downloading

DEG AM-resin (6.18 kg, 4.20 moles, 1.0 eq, 0.68 mmol/g) was added to the reactor. Then DMF (25 L, 50 - 58 °C) was added. After stirring for ≥ 20 minutes, the resin was drained and additional DMF (25 L, 50 - 58 °C) was added to the reactor and the mixture was stirred. For deprotonation of the resin, piperidine (2.5 L) was added and the mixture stirred for 15 minutes. Additional DMF was added and the reactor drained. The resin was washed batch-wise once with DMF. Then washed continuously with DMF until the wash solution showed a negative Chloranil test (indicating absence of piperidine in the wash solution) Finally, the resin was washed batch-wise with DMF once.

Fmoc-Ramage-OH linker and Oxyma (0.9 eq each, 3.78 moles) were dissolved in DMF (8 L) at 50 - 58 °C. The solution was added to the reactor containing deprotonated resin. Additional DMF (17 L) was added. Then DIC (2.25 eq) split in four portions was added in 5 minutes intervals to the reactor, total addition time 15 minutes, while maintaining the temperature at 50 - 58 °C. The three first portions contained each 13% of totally added DIC and the last portion 61%. The mixture was stirred for a total time of 45 minutes after addition of the first portion of DIC. Then extra DMF was added and the reactor drained. Finally, the resin was washed with DMF batch-wise twice.

AcOH and Oxyma (2 eq each, 8.40 moles) were dissolved in 8 L of DMF at 50 - 58 °C and added to the reactor. Additional DMF (17 L) was added and then DIC (5.0 eq) in four portions in 3 minutes intervals under stirring for a total time of 9 minutes; conditions otherwise as described above for coupling of the linker. After addition of the last portion of DIC, the reaction mixture was stirred for 6 min. Extra DMF was added and the reactor was drained. Finally, the resin was washed batch-wise with DMF once.

### 1.2 Attachment of Fmoc-Lys³⁹(Trt)-OH and second step of downloading via competitive co-capping

To the resin obtained in the previous step, DMF (25 L, 50 - 58 °C) was added with stirring. Then piperidine (2.5 L) was added to the reactor and the mixture was stirred for 20 min. The reactor was drained. The same amount of DMF was added and the treatment with piperidine was repeated. Then extra DMF was added and the reactor drained. Resin was washed batch-wise once with DMF. Then washed continuously with DMF until a negative Chloranil test was obtained. Then batch-wise washing of resin with DMF was carried out once.

Fmoc-Lys³⁹(Trt)-OH and Oxyma (1.5 eq each, 6.30 moles) were dissolved in 8 L of DMF at 50 - 58 °C and added to the reactor containing deprotected resin. Additional DMF (17 L) and AcOH (2.70 eq, 11.34 moles) were added to the reactor. Then DIC (3.75 eq) was added in four portions in four 5 minutes intervals under stirring while maintaining the temperature at 50 - 58 °C. The three first portions contained each 13% of the total amount of DIC and the last portion 61%. After addition of the last portion of DIC, the mixture was stirred for 30 minutes (total stirring time 45 minutes). Coupling was checked by the Kaiser test (negative test indicating no free amino groups on the resin) and additional DMF was added to the reactor. Then the reactor was drained and the resin was washed batch-wise with DMF once and drained.

### 1.3 Attachment of Fmoc-Lys³³(Trt)-OH, Fmoc-Lys³⁷(Boc)-OH, Fmoc-Lys³⁶(Boc)-OH, Fmoc-Lys³⁵(Boc)-OH, Fmoc-Lys³⁴(Boc)-OH

To the resin obtained in the previous step, DMF (25 L, 53 °C) was added with stirring. Then piperidine (625 ml) was added to the reactor and the mixture was stirred for 10 minutes. Thereafter piperidine (1875 ml) was added and the mixture was stirred for 10 min. Then the reactor was drained. The steps comprising deprotection with piperidine were repeated twice. After the last deprotection step, the mixture was diluted with DMF. Then the reactor was drained, and the resin washed batch-wise once with DMF. The resin was washed continuously with DMF until a negative Chloranil test was obtained. Then batch-wise washing of resin with DMF was carried out once.

Fmoc-protected amino acid and Oxyma (2.0 eq each, 4.20 moles) were dissolved in DMF (8 L) at 50 - 58 °C and added to the reactor containing deprotected resin. Additional DMF (17 L) was added and then DIC (5.0 eq) added portion-wise as described for attachment of the Ramage-linker **(Example 1.1).** After addition of the first portion of DIC, coupling was allowed to take place with stirring at 50 - 58 °C for a total time of 35 - 40 minutes. Coupling was checked by the Kaiser test. Then the resin was subjected to capping by addition of AcOH (2 eq, 5 moles) to the reaction and stirring for two minutes. Extra DMF was added and the reactor was drained. Finally, the resin was washed batch-wise with DMF once.

### 1.4 Attachment of Fmoc-Asp³³(OtBu)-OH

To the resin obtained in the previous step, DMF (25 L, 53°C) was added with stirring. Then piperidine (625 ml) was added to the reactor and the mixture was stirred for 5 minutes. Thereafter piperidine (1875 ml) was added to the reactor and the mixture was stirred for 5 minutes. Then the reactor was drained. The two-step deprotection was repeated, but with 10 minutes stirring after each piperidine addition. After the last deprotection step with piperidine, the mixture was diluted with DMF. Then the reactor was drained and the resin washed batch-wise once with DMF. The resin was washed continuously with DMF until a negative Chloranil test was obtained. Then batch-wise washing of resin with DMF was carried out once.

Fmoc-protected amino acid and Oxyma (2.0 eq each, 4.20 moles) were dissolved in DMF (8 L) at 50 - 58 °C and added to the reactor containing deprotected resin. Additional DMF (17 L) was added. Then DIC (5.0 eq) was added portion-wise to the reactor as described in **Example 1.1.** After addition of the first DIC portion coupling was allowed to take place at 50 - 58 °C with stirring for 35 - 40 minutes. Coupling was checked by the Kaiser test and the resin was subjected to capping with AcOH (2 eq) for 2 minutes and washings with DMF as described in **Example 1.3.**

### 1.5 Attachment of Fmoc-Thr³²(tBu)-OH, Fmoc-Thr²⁸(tBu)-OH, Fmoc-Ala²³-OH, Fmoc-Leu²¹-OH, Fmoc-Ile²³-OH, Fmoc-Arg²⁰(Pbf)-OH

Oxyma (711 g) was dissolved in DMF (8 L) at 50 - 58 °C and added to the reactor containing resin followed by DMF (17 L). Fmoc deprotection was performed by adding piperidine (625 ml) to the reactor containing Oxyma/DMF and resin and stirring the mixture for 5 minutes. Then piperidine (1875 ml) was added to the reactor and the mixture was stirred for 5 minutes. The reactor was drained. The two-step deprotection was repeated. After the last deprotection step with piperidine, the mixture was diluted with DMF. Then the reactor was drained and the resin washed batch-wise once. The resin was washed continuously with DMF until a negative Chloranil test was obtained. Then batch-wise washing of resin with DMF was performed once.

Fmoc-protected amino acid and Oxyma (2.0 eq each, 4.20 moles) were dissolved in DMF (8 L) at 50 - 58 °C and added to the reactor containing deprotected resin. Additional DMF (17 L) was added and DIC (5.0 eq) was added portion-wise to the reactor as described in **Example 1.1.** After addition of the first portion of DIC, the mixture was allowed to react with stirring at 50 - 58 °C for 30 minutes. Additional DMF was added and then the reactor was drained. Batch-wise washing of resin with DMF was carried out once.

The coupling step was repeated as described above. Except the reaction was allowed to take place for 35 - 40 min after addition of the first portion of DIC. Fmoc-Arg²⁰(Pbf)-OH was coupled a third time using 1 eq each of amino acid, Oxyma and DIC. Coupling was checked by the Kaiser test and the resin was subjected to capping with AcOH (2 eq) for 2 minutes as described in **Example 1.3.** Extra DMF was added and the reactor was drained. Finally, the resin was washed batch-wise with DMF once.

### 1.6 Attachment of Fmoc-Lys³⁰(Boc)-OH

The peptide resin was treated with Oxyma and piperidine as described in **Example 1.4** with the exception that two-step deprotection with stirring took place for 10 minutes after the first addition of piperidine and for 19 minutes after the second addition of piperidine.

Addition of Oxyma was repeated and the two-step deprotection with piperidine was performed by stirring resin for 10 minutes after each addition of piperidine. Additional DMF was added and then the reactor was drained. Batch-wise washing of resin with DMF was carried out once. The resin was washed continuously with DMF until a negative Chloranil test was obtained. Then batch-wise washing of resin with DMF was performed once.

Fmoc-protected amino acid and Oxyma (2.0 eq each, 4.2 moles) were dissolved in DMF (8 L) at 50 -58 °C and added to the reactor containing deprotected resin. Additional DMF (17 L) was added. Then DIC (5.0 eq) was added portion-wise to the reactor as described in **Example 1.1.** After addition of the first DIC portion coupling was allowed to take place at 50 - 58 °C with stirring for 35 - 40 minutes. Then coupling was checked by the Kaiser test and the resin was subjected to capping with AcOH (2 eq) for 2 minutes. Extra DMF was added and the reactor was drained. Finally, the resin was washed batch-wise with DMF once.

### 1.7 Attachment of Fmoc-Ser⁸(tBu)-OH and Fmoc-Thr⁵(tBu)-OH

Oxyma was added to the reactor as described in **Example 1.5.** Fmoc deprotection was performed by adding piperidine (625 ml) to the reactor containing Oxyma/DMF and resin and stirring the mixture for 10 minutes. Then piperidine (1875 ml) was added to the reactor and the mixture was stirred for 10 minutes. The reactor was drained. The two-step deprotection was repeated twice. After deprotection, the mixture was diluted with DMF. Then the reactor was drained and the resin washed batch-wise once with DMF. The resin was washed continuously with DMF until a negative Chloranil test was obtained. Then batch-wise washing of resin with DMF was performed once.

Fmoc-protected amino acid and Oxyma (2.0 eq each, 4.20 moles) were dissolved in DMF (8 L) at 50 - 58 °C and added to the reactor containing deprotected resin. Additional DMF (17 L) was added and DIC (5.0 eq) was added portion-wise to the reactor as described in **Example 1.1.** After addition of the first DIC portion coupling was allowed to take place at 50 - 58 °C with stirring for 35 - 40 minutes. Coupling was checked by the Kaiser test and the resin was subjected to capping with AcOH (2 eq) for 2 minutes. Extra DMF was added and the reactor was drained. Finally, the resin was washed batch-wise with DMF once.

### 1.8 Attachment of Fmoc-Gly⁴-OH

Oxyma was added to the reactor and deprotection of the Fmoc group by treatment with piperidine was performed as described in **Example 1.7** including application of the Chloranil test.

Fmoc-protected amino acid and Oxyma (2.0 eq each, 4.20 moles) were dissolved in DMF (8 L) at 50 - 58 °C and added to the reactor containing deprotected resin. Additional DMF (17 L) was added and DIC (5.0 eq) was added portion-wise to the reactor as described in **Example 1.1.** After addition of the first DIC portion, coupling took place with stirring for 30 minutes. Additional DMF was added and then the reactor was drained. Batch-wise washing of resin with DMF was carried out once.

The coupling step above was repeated and allowed to take place for 35 - 40 minutes after addition of the first DIC portion. Then coupling was checked by the Kaiser test and the resin was subjected to capping with AcOH (2 eq) for 2 minutes. Extra DMF was added and the reactor was drained. Finally, the resin was washed batch-wise with DMF once.

### 1.9 Attachment of Boc-His¹(Trt)-Gly²-OH

Oxyma (711 g) was dissolved in DMF (8 L) at 50 - 58 °C and added to the reactor containing resin. Additional DMF (17 L) was added. Fmoc deprotection was performed by adding piperidine (625 ml) to the reactor containing Oxyma/DMF and resin and stirring the mixture for 5 minutes. Then piperidine (1875 ml) was added to the reactor and the mixture was stirred for 5 minutes. The reactor was drained.

Addition of Oxyma was repeated and the two-step deprotection with piperidine was performed by stirring resin for 10 minutes after each addition of piperidine. Additional DMF was added and then the reactor was drained. Batch-wise washing of resin with DMF was carried out once. The resin was washed continuously with DMF until a negative Chloranil test was obtained. Then batch-wise washing of resin with DMF was performed once.

Boc-His¹(Trt)-Gly²-OH and Oxyma (1.5 eq each, 3.15 moles) were dissolved in DMF (8 L) at 50 - 58 °C and added to the reactor containing deprotected resin. Additional DMF (17 L) was added. Then DIC (3.75 eq) was added portion-wise to the reactor as described in **Example 1.1.** After addition of the first DIC portion coupling was allowed to take place at 50 - 58 °C with stirring for 60 minutes. Then coupling was checked by the Kaiser test. Extra DMF was added to the reactor and the reactor was drained. Finally, the resin was washed batch-wise with DMF once.

### 1.10 Attachment of remaining amino acids: Fmoc-Glu³(OtBu)-OH, Fmoc-Phe⁶-OH, Fmoc-Ser⁷(tBu)-OH, Fmoc-Glu⁹(OtBu)-OH, Fmoc-Leu¹⁰-OH, Fmoc-Ala¹¹-OH, Fmoc-Thr¹²(tBu)-OH, Fmoc-Ile¹³-OH, Fmoc-Leu¹⁴-OH, Fmoc-Asp¹⁵(OtBu)-OH, Fmoc-Ala¹⁶-OH, Fmoc-Leu¹⁷-OH, Fmoc-Ala¹⁸-OH, Fmoc-Ala¹⁹-OH, Fmoc-Asp²¹(OtBu)-OH, Fmoc-Phe²²-OH, Fmoc-Ala²¹-OH, Fmoc-Trp²⁵(Boc)-OH, Fmoc-Ile²⁷-OH, Fmoc-Ile³¹-OH

Oxyma treatment and deprotection with piperidine were performed as described in **Example 1.9.** After deprotection, additional DMF was added. Then the reactor was drained. Batch-wise washing of resin with DMF was carried out once. The resin was washed continuously with DMF until a negative Chloranil test was obtained. Then batch-wise washing of resin with DMF was performed once.

Fmoc amino acid and Oxyma (2.0 eq each, 4.20 moles) were dissolved in DMF (8 L) at 50 - 58 °C and added to the reactor containing deprotected resin. Additional DMF (17 L) was added to the reactor and DIC (5.0 eq) was added as described in **Example 1.1.** After addition of the first DIC portion coupling was allowed to take place at 50 - 58 °C with stirring for 35 - 40 minutes. Then coupling was checked by the Kaiser test and the resin was subjected to capping with AcOH (2 eq) for 2 minutes. Extra DMF was added and the reactor was drained. Finally, the resin was washed batch-wise with DMF once.

### 1.11 Deprotection and cleavage of peptide from resin

After completed synthesis, protected peptide resin was washed batch-wise three times with DMF, twice with DMF at 50 - 58°C, and once with DMF at room temperature. Then the peptide resin was washed 5 times with isopropanol. Peptide resin was finally dried at 25 - 35°C and stored at 2 - 8°C.

TFA (68.1 kg), DTT (1.75 L), TIS (1.25 L), and water (1.25 L) was added to the reactor. Peptide resin (10 kg corresponding to 0.8 mole) prepared as described in **Examples 1.1-1.10** was added to the solution. The mixture obtained was left with stirring at 25°C for 135 minutes. Then the temperature was decreased to less than 0°C and cold (< 0°C) MTBE (150 L) was slowly added to the reactor. During addition of MTBE, the temperature was kept ≤ 10°C while the peptide cleaved from resin precipitated. After complete precipitation, the mixture was left under stirring for 45±15 minutes at < 10°C. Then the mixture was filtered and washed with MTBE twice and once with a solution containing a mixture of MTBE (3 volumes) and acetonitrile (1 volume). After filtration, the filter cake obtained was dried under vacuum for 1 - 3 hrs.

### 1.12 Pre RPC1 (O → N acyl shift)

Conventionally, the O → N acyl shift is performed in solution using the following procedure. To the dried resin, a solution containing AcOH/MeCN/ H₂O + 1% NH₄OAc (w/w) (10%/50%/40%) was added. The mixture was left with stirring overnight. Then the mixture was filtered and the filter cake was washed with the same solution. The combined filtrates from two cleavages containing crude peptide (maximum 3600 g, equivalent to about 0.8 moles of peptide) were stored at 5°C.

According to methods of the present invention, the O → N acyl shift is performed on a column as described herein, in particular at Example 3.

### 1.13 Purification RPC1 (first dimension)

Buffers used in RPC1:
- Buffer A: 0.1% H₃PO₄
- Buffer B: MeCN
- Buffer C: 45 mM H₃PO₄, pH 2.2 + 100 mM NaCl
- Buffer D: 45 mM H₃PO₄, pH 7.7-8.0 + 100 mM NaCl

The solution from the deprotection and cleavage step was diluted to 5 times its volume with 0.2 M ammonium acetate in water. The solution was filtered and a maximum of 15 g/L column volume was applied to a column packed with C18 silica gel (column dimensions: 45x(50-45 cm)) preequilibrated with a solution containing 95% C and 5% B. After application of the peptide solution, the column was washed with the equilibration solution and then with a solution containing 95% D and 5% B until pH of the eluate was > 7.5. Prior to elution, the column was washed with a solution containing 95% C and 5% B until pH was < 2.5. (Pre-RPC1.)

Then the column was washed with a solution containing 93% A and 7% B. Adsorbed peptide was eluted by application of a gradient: mobile phase changed from 7% B (93% A) to 22% B (78% A) in 12 column volumes. The gradient was held at 22% B (78% A) Thereafter isocratic elution with 22% B until UV of the eluted peak reached 40-45%. Elution was monitored at 280 nm and fractions collected were analyzed by RP-HPLC. This process was repeated for purification of the remaining pool from the deprotection and cleavage step, and fractions showing a peptide purity ≥ 85% were pooled. Fractions showing a purity ≥ 55% and ≤ 85% were re-chromatographed by applying a maximum of 15 g/L column volume on the C18 column after dilution of the combined fractions with water to twice the volume. Fractions from the primary runs collected before the main peak were eluted by application of a gradient: mobile phase changed from 10% B (90% A) to 14% B (86% A) during 1 column volume and then 14% B (86% A) to 23% B (77% A) in 9 column volumes and thereafter isocratic elution with 23% B (77% A) until UV of the eluted peak reached 95%.

Fractions from the primary runs collected after the main peak were eluted by application of a gradient: mobile phase changed from 10% B (90% A) to 14% B (86% A) during 1 column volume and then 14% B (86% A) to 22% B (78% A) in 9 column volumes and thereafter isocratic elution with 22% B (78% A) until UV of the eluted peak reached 90%. Fractions after re-chromatography with purity ≥ 85% were mixed with the main pool, yielding the final pool of RPC1. Thus for purification of 3.6 kg crude peptide, the process described included three primary runs and one front and one back re-run, yielding the final RPC1 pool.

### 1.14 Purification RPC2 (second dimension)

Buffers used in RPC2:
- Buffer A: 0.1% TFA
- Buffer B: MeCN

The final pool from RPC1 was diluted with water to double the volume and a maximum of 13 g/L column volume was applied to a RPC column packed with C18 silica gel (column dimensions: 45x(50-45 cm)), preequilibrated with a solution containing 90% A and 10% B. After application of the peptide, the column was washed with five column volumes of the equilibration solution. Adsorbed peptide was eluted with a gradient: mobile phase changed from 10% B (90% A) to 19% B (81% A) during 1 column volume and then from 19% B (81% A) to 30% B (70% A) during 12 column volumes. Thereafter isocratic elution until UV of the eluted peak reached 40-45% of its maximum value. The gradient was then changed to 60% B (40% A) and held at this value until all peptide was eluted. Elution was monitored at 280 nm and fractions collected were analyzed by RP-HPLC. This process was repeated for purification of the remaining pool from RPC1 and fractions showing a purity ≥ 92 and impurity des-Ile²⁷-Ala²⁸< 0.5% were pooled. After dilution with water, fractions collected before the main peak showing a purity > 50% and < 92% and/or impurity des-Ile²⁷-Ala²⁸ > 0.5% < 1.5% and fractions collected after the main peak, showing a purity > 50% and < 92% were re-chromatographed by applying a maximum of 20 g/L column volume on the C18 column and eluted by application of a gradient: mobile phase changed from 10% B (90% A) to 20% B (80% A) during 1 column volume and then 20% B (80% A) to 30% B (70% A) in 9 column volumes and thereafter isocratic elution with 30% B (70% A) until UV of the eluted peak reached 45%.

Fractions obtained after re-chromatography with a purity ≥ 92% impurity and des-Ile²⁷-Ala²⁸ < 0.5% were mixed with the main pool, yielding the final pool of RPC2.

### 1.15 Purification RPC3 (third dimension)

Buffers used in RPC3:
- Buffer A: 100 mM NH₄OAc + 0.5% AcOH
- Buffer B: MeCN

The final pool from RPC2 was diluted with water to double the volume and a maximum of 13 g/L column volume was applied to a RPC column packed with C18 silica gel (column dimensions: 45x(50-45 cm)), preequilibrated with a solution containing 85% A and 15% B. After application of the peptide, the column was washed with the equilibration solution. Adsorbed peptide was eluted with a gradient: mobile phase changed from 15% B (85% A) to 29% B (71% A) during 1 column volume and then from 29% B (71% A) to 37% B (63% A) during 10 column volumes. Thereafter isocratic elution until UV-signal reached 30% of its maximum value. The gradient was then changed to 70% B (30% A) and held at this value until the UV-signal reached the baseline. Elution was monitored at 280 nm and the pH of the fractions collected was adjusted to 5.8-6.0 with aqueous ammonia. Fractions collected were analyzed by RP-HPLC and those showing a peptide purity ≥ 96.5% and no single impurity before the main peak > 0.5%, were pooled, yielding the final main pool of RPC3. This process was repeated for purification of the remaining pool from RPC2.

### 1.16 Purification RPC4 (fourth dimension)

Buffers used in RPC4:
- Buffer A: 5 mM NH₄OAc + 0.1% AcOH
- Buffer B: MeCN
- Buffer C: 100 mM NH₄OAc + 0.5% AcOH

The final pool from RPC3 was diluted with water to double the volume and ammonium acetate was added to the solution obtained to give a final concentration of about 100 mM ammonium acetate in the pool. A maximum of 15 g/L column volume was applied to a RPC column packed with Amberchrom XT20 (column dimensions: 45x(40-35 cm)), preequilibrated with 90% C and 10% B. After application of the peptide, the column was washed with one column volume of the equilibration solution and one column volume of 90% A and 10% B. Adsorbed peptide was eluted with a gradient: mobile phase changed from 10% B (90% A) to 13% B (87% A) during 1 column volume and then from 13% B (87% A) to 25% B (75% A) during 12 column volumes. When the UV-signal reached 40% of its maximum value, the gradient was then changed to 60% B (40% A) and held at this value until all peptide was eluted. Elution was monitored at 280 nm and the pH of the fractions collected was adjusted to 5.8-6.0 with aqueous ammonia. The process was repeated for purification of the remaining pool from RPC3. Fractions collected were analyzed by RP-HPLC and fractions showing a peptide purity ≥ 98.0% and no individual impurity > 0.5% were pooled. After dilution with water, fractions collected before the main peak showing a purity ≥ 80.0% and < 98.0% and/or impurity Des-Ser^{7/8}/Aspartimide > 0.5% and fractions collected after the main peak, showing a purity > 80.0% and < 98.0% and/or impurity Des-Ser^{7/8}/Aspartimide > 0.5% were re-chromatographed by applying a maximum of 15 g/L column volume on the Amberchrom XT20 column. The product was eluted by application of a gradient: mobile phase changed from 10% B (90% A) to 13% B (87% A) during 1 column volume and then 13% B (87% A) to 25% B (75% A) in 9 column volumes and thereafter isocratic elution with 25% B (75% A) until UV of the eluted peak reached 30%. Fractions were pH adjusted to 5.8-6.0 with aqueous ammonia and analyzed by RP-HPLC. Pools obtained after the re-chromatography with a purity ≥ 98.0%, and no individual impurities > 0.5% were mixed with the main pool, yielding the final pool of RPC4.

### 1.17 Desalting (SPE)

Buffers used for desalting:
- Buffer A: 10 mM AcOH
- Buffer B: MeCN

The final pool from RPC4 was diluted by addition of water to double the volume and then ammonium acetate was added to give a final concentration of about 100 mM in the pool. The pool was applied to the column packed with Amberchrom XT20 (column dimensions: 45x40 cm), preequilibrated with 95% A and 5% B. After application of the peptide, the column was washed with the equilibration solution. Adsorbed peptide was eluted with a gradient: mobile phase changed from 5% B (95% A) to 50% B during 1 column volume. The gradient was held at this value until all peptide was eluted. Elution was monitored at 280 nm and the fractions collected were analyzed by RP-HPLC.

### 1.18 Isolation of purified peptide

The product from the desalting step was subjected to evaporation under reduced pressure at ≤ 40°C. On this treatment, MeCN was evaporated and the peptide solution was reduced to roughly 30% of the initial volume and then diluted with water yielding a final peptide concentration of about 25 g/L. The concentrated peptide was filtered through a 0.45/0.22 µm filter and then isolated via lyophilization, yielding about 1.3 kg of purified peptide (total purification yield ≥ 35%) showing a purity of ≥ 97.75% and no single impurity > 0.5% by RP-HPLC.

### Example 2: Optimization of the crude peptide by use of Fmoc-Lys(Trt)-OH

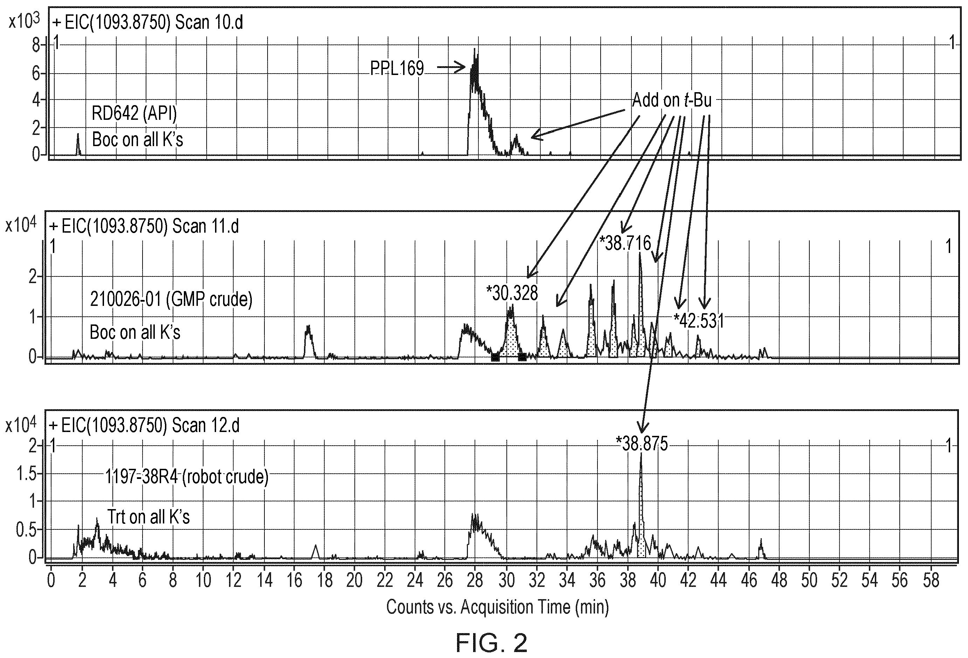

During development of the ZP1848 synthesis, it was noted that the presence of Lys(Boc)₆ did, as might be expected, lead to a number of tert-butylated by-products (+56 Da). Particularly some of these eluted close to the main peak, and hence could also be found in the purified solution **(****Figure 2****,** top panel). Without wishing to be bound by any theory, the inventors believe that this +56 impurity is related to the cleavage of a *tert-*butyl group and hence the +56 moiety is thought to be located somewhere on the lysine tail, most likely on 37 or 38.

Performing the ZP1848 synthesis with [Lys(Trt)]₆ eliminated to a high degree *tert*-butylated by-products **(****Figure 2****,** bottom panel), although some butylated by-products may nonetheless be observed as a result of transfer from protecting groups elsewhere in the synthesis.

It is not necessary that Lys(Trt) is used for each lysine residue in the tail, although it may be. Since Fmoc-Lys(Trt)-OH is a more expensive building block than Fmoc-Lys(Boc)-OH, particularly on a mole basis, a cost-efficient process may only apply this building block to only some of the amino acid couplings.

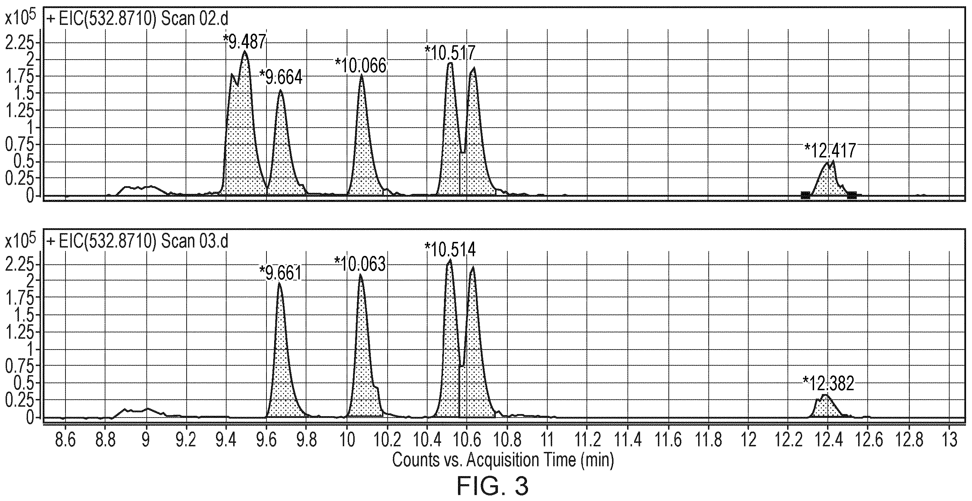

Furthermore, the inventors have observed that the *tert*-butylated by-products Lys³⁹-*t*-Bu and Lys³⁸-*t*-Bu, derived from using Lys³⁹(Boc) and Lys³⁸(Boc) residues, are the most difficult impurities to remove from the crude peptide to obtain the desired peptide. Changing the protecting group from Boc to Trt for these two residues prevents the formation of the Lys³⁹-*t-*Bu and Lys³⁸-*t*-Bu *tert*-butylated by-products. Accordingly, the inventors have determined that using Lys(Trt) for only the first two couplings (that is, for Lys³⁹ and Lys³⁸ residues), giving a [K(Boc)]₄[K(Trt)]₂ motif, facilitates elimination of the *tert*-butylated by-products eluting closest to the main peak **(****Figure 3****,** peak at 9.487), and hence, allows for a purer crude product. This, in turn, allows for a cleaner purification process giving a higher yield and a purer final product.

### Example 3: O to N acyl shift on column in Pre-RPC1

During cleavage of ZP1848 **(Example 1.11)** an N → O acyl shift takes place. Acetylated (that is, truncations of the peptide chain) and trifluoro-acetylated impurities (that is, trifluoro-acetyl esters of the peptide chain) are also found, when the crude, precipitated product is dissolved in a mixture of AcOH/MeCN/1% aqueous NH₄OAc 1:5:4 and left overnight for decarboxylation of Trp(Boc).

It is known that adjusting pH to neutral and back to acidic conditions prior to the purification steps reduced these impurities, thus performing an O → N acyl shift. See, for example, (a) Bergmann M, Brand E, Weimann F. Z Physiol Chem. 1923;131:1-17; (b) Phillips AP, Baltzly R. J Am Chem Soc. 1947;69:200-204.

The O → N acyl shift is usually performed in solution before the peptide product is added to the column. In order to perform the shift, the pH is normally reduced using phosphate buffer/phosphoric acid.

However, it was found that when using phosphate buffer/phosphoric acid in the peptide product solution, ZP1848 precipitates at neutral pH (for further details concerning the phosphate buffer incompatibility, see example 4 of WO2020/065064). It was further found that it is not feasible to raise the pH to neutral pH without precipitation due to the high content of acetic acid in the peptide product solution prior to purification as that would require a very large amount of NaOH which is very impractical.

Additionally, ZP1848 precipitates at neutral pH in solutions containing NaCl, phosphate, NaOH and high ionic strength.

The purpose with this Example is to compare the results when performing the O → N acyl shift in solution and on the column.

Below in **Table 4.1** various formulations of ZP1848 were prepared in 100 mM NaCl and 45 mM phosphate (the buffers are described in **Example 1.12)** at different pH and/or concentrations in order to reflect the same conditions in solution as present in the column under RPC1.

**Table 4.1: Various peptide product solutions comprising phosphate**

| **Formulation No.** | **Concentration [mg/mL] of ZP1848** | **pH** | **Precipitation observed (yes or no)** |
|---|---|---|---|
| **1** | 10 | 2.2 | no |
| **2** | 2 | 2.2 | no |
| **3** | 10 | 7.5 | yes |
| **4** | 2 | 7.5 | yes |
| **5** | 10 | 7.0 | yes |
| **6** | 2 | 7.0 | no |
| **7** | 0.2 | 7.5 | yes |
| **8** | 0.5 | 7.5 | yes |

The data in **Table 4.1** clearly demonstrate that ZP1848 precipitates when using the same conditions in solution as used on the column during RPC1. The precipitations occur within minutes after the sample preparations. Additionally, it was shown that ZP1848 precipitates even at low concentrations at pH 7.5 (0.2 and 0.5 mg/mL).

It has surprisingly been found that is possible to perform this pH treatment on the C18 column used for purification prior to the first purification step. It is especially surprising that a phosphate buffer can be used without loss of material. In practice, as Pre-RPC1 and RPC1 are performed on the same column, this removes a processing step from the purification sequence.

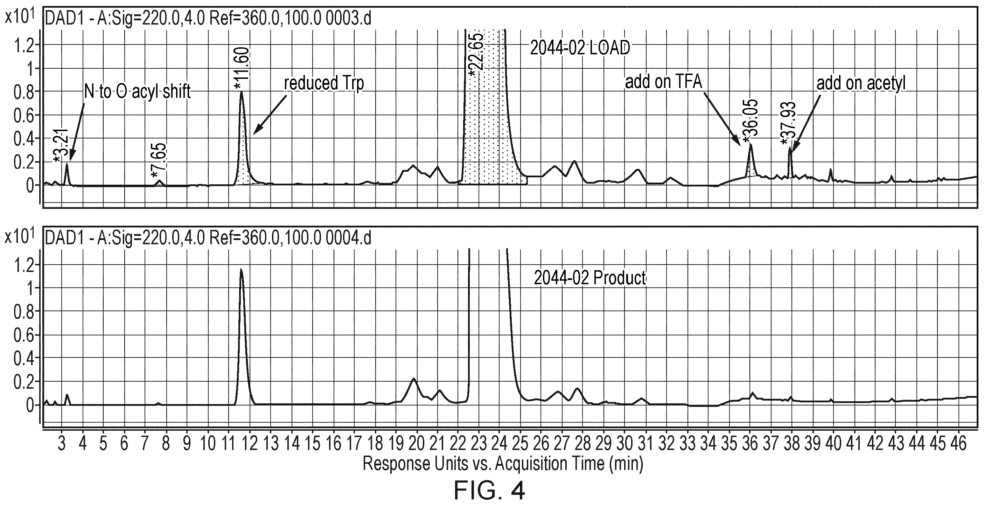

In the cleavage, the data in **Figure 4** show that the TFA and acyl impurities, as well as the acyl shift is lowered after pH treatment on the column. Furthermore, the possible yield lost on the column due to the peptide being incompatible with phosphate buffer at neutral pH (possible precipitation) was also evaluated. In the experiment in **Figure 4****,** 100 mg was used before pH treatment and after pH treatment, the amount of ZP1848 was quantified to 89 mg (the loss of ca. 10 mg can be explained by handling of product during cleavage/precipitation). Therefore, the loss on the column was considered negligible and no yield lost has been observed due to the pH treatment on column. In conclusion, performing the O → N acyl shift on column prevents an undesired precipitation while achieving the desired chemical transformations. Furthermore, by raising pH to neutral, O → N acyl shift and hydrolysis of the acetylated and trifluoro-acetylated impurities increases the yield by approx. 5%. That is, the quantified amount of product eluting from the RPC1 step is approx. 5% higher than the quantified amount in the load.

### Example 4: Purification of crude peptide and removal of unwanted components

As outlined in **Example 1,** using a five-step (a "pre" step and four "dimensions") chromatographic purification process (Pre RPC1 + RPC1-RPC4) of the Fmoc-SPPS resulted in a ZP1848 product with a purity of 98.2%. It was found that the ZP1848 product contained not more than 0.5% of individual impurities, when assessed with analytical HPLC with UV or MS detection. Each step of the purification process was developed for the removal of particular unwanted components such as peptide impurities (i.e. high molecular weight (HMW) truncations, deletions, or other undesired derivatives) or to improve purity of the final substance. These unwanted components may comprise of covalent or non-covalent impurities where the physiological activity is altered, inactivated or unknown side effects and should be reduced as much as possible. In the ZP1848 product, at least one species of C-terminal deamidation, HMW compounds, and formation of Aspartic acid related impurities (such as formation of Iso-Asp/Beta-Asp and Aspartimide) from aspartic acid, are particularly undesired impurities.

### Removal of specific impurities - C-terminal deamidation product, Lys³⁹-OH impurity and aspartic acid related impurities

The order of the RPC steps is crucial insofar that RPC1 needs to be performed before RPC2 and 3 because RPC1 removes impurities that otherwise will obscure details that are essential to be seen in these two steps.

In the first dimension (RPC-1) a phosphate buffer was used, in the second TFA, and third and fourth ammonium acetate. Acetonitrile (MeCN) was used as organic modifier for all purification steps. In order to evaluate purification efficacy, fractions after each step of the main component were analysed by either HPLC or LC-MS. The results of the obtained purity, main undesired impurities and particular, hard to remove impurities are listed in **Table 4.1** below. It can be seen that through sequential purification steps, aspartic acid-related impurities (formation of Iso-Asp/Beta-Asp and aspartimide) are reduced to achieve a final product purity of ≥98%.

**Table 4.1**

| **Sample** | **C-terminal deamidation** (Lys³⁹-OH impurity) | **Aspartic acid related impurities** | **Purity** |
|---|---|---|---|
| Crude | 2.9% | >10% | ~45% |
| After RPC1 | 0% | 8.4% | 89% |
| After RPC2 | 0% | 1.7% | 96% |
| After RPC3 | 0% | 0.9% | 98% |
| After RPC4 | 0% | 0.5% | ≥98% |

The first RPC is used for isolation of the main component and for removal of specific impurities, i.e., aspartic acid related impurities and C-terminal deamidation impurities. Most truncated terminal amino acid moieties are also removed in this step. In the crude before RPC1 the level of the C-terminal deamidation product, Lys³⁹-OH impurity is relatively high 2.9% and after running RPC1 it elutes only in the last fraction making this purification step very efficient for the removal of this impurity. If RPC2 is used instead the C-terminal deamidation product, Lys³⁹-OH impurity elutes over 5 fractions containing over 50% ZP1848 as well. This shows that RPC1, and the use of phosphate buffer, is able to reduce this impurity very efficiently, whereas RPC2 would have failed as well as compromising the overall yield and purity.

The use of phosphate buffer was particularly good to be used for this purpose whereas TFA and ammonium acetate are unsuccessful in separating the C-terminal deamidation product from the main product. The second, third and fourth step improved purity but is also used for removal of aspartic acid related impurities.

### Removal of oligomer

Oligomers are mainly generated before purification and are observed in the crude solution (cleavage acidic conditions). **Table 4.2** shows content of oligomers throughout the purification of ZP1848. The level of covalently bonded oligomeric products is 1.8% in the crude solution, but after the O → N acyl shift there is a slight increase to 2.4%. The oligomers are removed primarily in RPC1 and partly in RPC2 and in the final product the level is below 0.1%. The data also shows that there is no further generation of oligomers throughout the purification process.

**Table 4.2: Content of oligomers throughout the purification process of ZP1848 for a representative batch**

| **Sample ID** | **SEC results [Area % oligomer]** |
|---|---|
| Crude | 1.8 |
| Before RPC1 | 2.4 |
| Before RPC2 | 0.36 |
| Before RPC3 | < 0.05 |
| API | ~0.07 |

As can be seen from **Table 4.2,** after RPC1 the oligomers are strongly reduced and after RPC2 they are present in only minute (i.e. small) amounts. The amount of oligomers is not expected to increase during the pH treatment on the column.

## Claims

1. A method of producing a GLP-2 analogue, wherein the GLP-2 analogue is represented by the formula:
R¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R²
wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z² is absent or a peptide sequence of 1-6 amino acid units of Lys;
or a pharmaceutically acceptable salt or derivative thereof;
the method comprising the steps of:
i) loading a C8 or C18 column with the crude GLP-2 analogue synthesized by solid phase peptide synthesis (SPPS);
ii) adjusting the pH of the column with a first buffer system, wherein the first buffer system is a phosphate buffer system;
wherein step ii) includes sequential use of said buffer system to raise the pH of the column to greater than 7.5 while the GLP-2 analogue is on the column followed by lowering the pH of the column to an acidic pH.

2. The method of claim 1, wherein Z² is a peptide sequence of 1-6 amino acid units of Lys.

3. The method of any one of the preceding claims, wherein the method further comprises thereafter:
(1) i) eluting a pool containing the GLP-2 analogue using said first buffer system; then
(2) i) loading a column with the pool containing the GLP-2 analogue obtained in step (1); and
ii) washing the column with a second buffer system to elute a pool containing the GLP-2 analogue;
wherein the second buffer system includes trifluoroacetic acid; then
(3) i) loading a column with the pool containing the GLP-2 analogue obtained in step (2); and
ii) washing the column with a third buffer system to elute a pool containing the GLP-2 analogue;
wherein the third buffer system includes acetic acid/ammonium acetate; then
(4) i) loading a column with the pool containing the GLP-2 analogue obtained in step (3); and
ii) washing the column with a fourth buffer system to elute a pool containing the GLP-2 analogue;
wherein the fourth buffer system includes acetic acid/ammonium acetate.

4. The method of any one of the preceding claims, further comprising the method of synthesising the GLP-2 analogue by solid phase peptide synthesis (SPPS), wherein Z² is a peptide sequence of 1-6 amino acid units of Lys and wherein at least one of the Lys units in Z² is protected with a trityl protecting group during the synthesis.

5. The method of claim 4, wherein Z² is a peptide sequence of 2-6 amino acid units of Lys and the method comprises the steps of:
i) attaching a first P-Lys(Trt)-OH to a solid-state peptide resin with a linker;
ii) removing the P group from the Lys(Trt) amino acid unit;
iii) attaching a second P-Lys(Trt)-OH to the Lys(Trt) amino acid unit attached to the solid-state peptide resin with a linker;
iv) removing the P group from the second Lys(Trt) amino acid unit;
v) attaching subsequent amino acid units until the GLP-2 analogue is synthesized;
vi) cleaving the GLP-2 analogue from the solid-state peptide resin; and
vii) purifying the GLP-2 analogue.
where each P is a protecting group.

6. The method of any one of the preceding claims, wherein X5 is Thr and/or X11 is Ala.

7. The method of claim 6, wherein the GLP-2 analogue is selected from:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); and
ZP2530 H-HGEGTFSSELATILDALAARDFIAWLIATKITD-OH (SEQ ID NO: 6).

8. The method of claim 6, wherein the GLP-2 analogue is selected from:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3); and
ZP2469 H-HGEGTFSSELATILDALAARDFIAWLIATKITDK-OH (SEQ ID NO: 4).

9. The method of claim 6, wherein the GLP-2 analogue is:
ZP1848 H-HGEGTFSSELATILDALAARDFIAWLIATKITDKKKKKK-NH₂ (SEQ ID NO: 1).

10. The method of any of claims 1 to 5, wherein X5 is Ser and/or X11 is Ser.

11. The method of claim 10, wherein the GLP-2 analogue is selected from:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); and
ZP2242 H-HGEGSFSSELSTILDALAARDFIAWLIATKITDK-OH (SEQ ID NO: 9).

12. The method of claim 10, wherein the GLP-2 analogue is:
ZP1846 H-HGEGSFSSELSTILDALAARDFIAWLIATKITDKKKKKK-NH₂ (SEQ ID NO: 7).

## Patentansprüche

1. Verfahren zur Herstellung eines GLP-2-Analogons, wobei das GLP-2-Analogon durch folgende Formel dargestellt ist:
R¹-His-Gly-Glu-Gly-X5-Phe-Ser-Ser-Glu-Leu-X11-Thr-Ne-Leu-Asp-Ala-Leu-Ala-Ala-Arg-Asp-Phe-Ile-Ala-Trp-Leu-Ile-Ala-Thr-Lys-Ile-Thr-Asp-Z²-R²
worin:
R¹ Wasserstoff, C₁₋₄-Alkyl (z. B. Methyl), Acetyl, Formyl, Benzoyl oder Trifluoracetyl ist;
X5 Ser oder Thr ist;
X11 Ala oder Ser ist;
R² NH₂ oder OH ist; und
Z² fehlt oder eine Peptidsequenz von 1 bis 6 Aminosäureeinheiten von Lys ist;
oder ein pharmazeutisch annehmbares Salz oder Derivat davon;
wobei das Verfahren die folgenden Schritte umfasst:
i) Beladen einer C8- oder C18-Säule mit dem rohen GLP-2-Analogon, das durch Festphasen-Peptidsynthese (SPPS) synthetisiert wurde;
ii) Einstellen des pH-Werts der Säule mit einem ersten Puffersystem, wobei das erste Puffersystem ein Phosphatpuffersystem ist;
wobei Schritt ii) die anschließende Verwendung des Puffersystems zum Erhöhen des pH-Werts der Säule auf über 7,5 umfasst, während sich das GLP-2-Analogon auf der Säule befindet, gefolgt vom Erniedrigen des pH-Werts der Säule auf einen sauren pH-Wert.

2. Verfahren nach Anspruch 1, wobei Z² eine Peptidsequenz aus 1 bis 6 Aminosäureeinheiten von Lys ist.

3. Verfahren nach einem der vorangegangenen Ansprüche, wobei das Verfahren weiters danach Folgendes umfasst:
(1) i) Eluieren eines Pools, der das GLP-2-Analogon enthält, unter Verwendung des ersten Puffersystems;
(2) i) Beladen einer Säule mit dem Pool, der das GLP-2-Analogon enthält, der in Schritt (1) erhalten wurde; und
ii) Waschen der Säule mit einem zweiten Puffersystem, um einen Pool, der das GLP-2-Analogon enthält, zu eluieren;
wobei das zweite Puffersystem Trifluoressigsäure umfasst; dann
(3) i) Beladen einer Säule mit dem in Schritt (2) erhaltenen Pool, der das GLP-2-Analogon enthält; und
ii) Waschen der Säule mit einem dritten Puffersystem, um einen Pool, der das GLP-2-Analogon enthält, zu eluieren;
wobei das dritte Puffersystem Essigsäure/Ammoniumacetat umfasst; dann
(4) i) Beladen einer Säule mit dem in Schritt (3) erhaltenen Pool, der das GLP-2-Analogon enthält; und
ii) Waschen der Säule mit einem vierten Puffersystem, um einen Pool, der das GLP-2-Analogon enthält, zu eluieren;
wobei das vierte Puffersystem Essigsäure/Ammoniumacetat umfasst.

4. Verfahren nach einem der vorangegangenen Ansprüche, das weiters ein Verfahren zum Synthetisieren des GLP-2-Analogons durch Festphasenpeptidsynthese (SPPS) umfasst, wobei Z² eine Peptidsequenz von 1 bis 6 Aminosäureeinheiten von Lys ist und wobei zumindest eine der Lys-Einheiten in Z² während der Synthese mit einer Tritylschutzgruppe geschützt ist.

5. Verfahren nach Anspruch 4, wobei Z² eine Peptidsequenz von 2 bis 6 Aminosäureeinheiten von Lys ist und das Verfahren die folgenden Schritte umfasst:
i) Binden eines ersten P-Lys(Trt)-OH an ein Festkörperpeptidharz mit einem Linker;
ii) Entfernen der P-Gruppe aus der Lys(Trt)-Aminosäureeinheit;
iii) Binden eines zweiten P-Lys(Trt)-OH an die Lys(Trt)-Aminosäureeinheit, die mit einem Linker an das Festkörperpeptidharz gebunden ist;
iv) Entfernen der P-Gruppe aus der zweiten Lys(Trt)-Aminosäureeinheit;
v) Binden von darauffolgenden Aminosäureeinheiten, bis das GLP-2-Analogon synthetisiert ist;
vi) Abspalten des GLP-2-Analogons von dem Festkörperpeptidharz; und
vii) Reinigen des GLP-2-Analogons,
wobei die P jeweils eine Schutzgruppe sind.

6. Verfahren nach einem der vorangegangenen Ansprüche, wobei X5 Thr ist und/oder X11 Ala ist.

7. Verfahren nach Anspruch 6, wobei das GLP-2-Analogon aus Folgenden ausgewählt ist:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3);
ZP2469 H-HGEGTFSSELATILDALAARDFIAWLIATKITDK-OH (SEQ ID NO: 4);
ZP1857 H-HGEGTFSSELATILDALAARDFIAWLIATKITD-NH₂ (SEQ ID NO: 5); und
ZP2530 H-HGEGTFSSELATILDALAARDFIAWLIATKITD-OH (SEQ ID NO: 6).

8. Verfahren nach Anspruch 6, wobei das GLP-2-Analogon aus Folgenden ausgewählt ist:
ZP1848 H-HGEGTFSSELATILDALAARDFIAWLIATKITDKKKKKK-NH₂ (SEQ ID NO: 1)
ZP2949 H-HGEGTFSSELATILDALAARDFIAWLIATKITDKKK-OH (SEQ ID NO: 2);
ZP2711 H-HGEGTFSSELATILDALAARDFIAWLIATKITDKK-OH (SEQ ID NO: 3); und
ZP2469 H-HGEGTFSSELATILDALAARDFIAWLIATKITDK-OH (SEQ ID NO: 4).

9. Verfahren nach Anspruch 6, wobei das GLP-2-Analogon Folgendes ist:
ZP1848 H-HGEGTFSSELATILDALAARDFIAWLIATKITDKKKKKK-NH₂ (SEQ ID NO: 1).

10. Verfahren nach einem der Ansprüche 1 bis 5, wobei X5 Ser ist und/oder X11 Ser ist.

11. Verfahren nach Anspruch 10, wobei das GLP-2-Analogon aus Folgenden ausgewählt ist:
ZP1846 H-HGEGSFSSELSTILDALAARDFIAWLIATKITDKKKKKK-NH₂ (SEQ ID NO: 7);
ZP1855 H-HGEGSFSSELSTILDALAARDFIAWLIATKITD-NH₂ (SEQ ID NO: 8); und
ZP2242 H-HGEGSFSSELSTILDALAARDFIAWLIATKITDK-OH (SEQ ID NO: 9).

12. Verfahren nach Anspruch 10, wobei das GLP-2-Analogon Folgendes ist:
ZP1846 H-HGEGSFSSELSTILDALAARDFIAWLIATKITDKKKKKK-NH₂ (SEQ ID NO: 7).

## Revendications

1. Procédé de production d'un analogue de GLP-2, dans lequel l'analogue de GLP-2 est représenté par la formule :
R¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R²
dans lequel :
R¹ est un atome d'hydrogène, un alkyle en C₁₋₄ (par exemple, un méthyle), un acétyle, un formyle, un benzoyle ou un trifluoroacétyle ;
X5 est Ser ou Thr ;
X11 est Ala ou Ser ;
R² est un NH₂ ou un OH ;et
Z² est absent ou une séquence peptidique de 1 à 6 unités d'acides aminés de Lys ;
ou un sel ou un dérivé pharmaceutiquement acceptable de celui-ci ;
le procédé comprenant les étapes consistant à :
i) charger une colonne C8 ou C18 avec l'analogue de GLP-2 brut synthétisé par synthèse peptidique en phase solide (SPPS) ;
ii) ajuster le pH de la colonne avec un premier système tampon, dans lequel le premier système tampon est un système tampon phosphate ;
dans lequel l'étape ii) inclut l'utilisation séquentielle dudit système tampon pour élever le pH de la colonne à plus de 7,5 pendant que l'analogue du GLP-2 est sur la colonne, suivie de l'abaissement du pH de la colonne à un pH acide.

2. Procédé selon la revendication 1, dans lequel Z² est une séquence peptidique de 1 à 6 unités d'acides aminés de Lys.

3. Procédé selon l'une quelconque des revendications précédentes, dans lequel le procédé comprenant en outre, par la suite, les étapes consistant à :
(1) i) éluer un pool contenant l'analogue de GLP-2 en utilisant ledit premier système tampon ;
(2) i) charger une colonne avec le pool contenant l'analogue de GLP-2 obtenu à l'étape (1) ; et
ii) laver la colonne avec un deuxième système tampon pour éluer un pool contenant l'analogue de GLP-2 ;
dans lequel le deuxième système tampon inclut de l'acide trifluoroacétique ; puis
(3) i) charger une colonne avec le pool contenant l'analogue de GLP-2 obtenu à l'étape (2) ; et
ii) laver la colonne avec un troisième système tampon pour éluer un pool contenant l'analogue de GLP-2 ;
dans lequel le troisième système tampon inclut de l'acide acétique/acétate d'ammonium ; puis
(4) i) charger une colonne avec le pool contenant l'analogue de GLP-2 obtenu à l'étape (3) ; et
ii) laver la colonne avec un quatrième système tampon pour éluer un pool contenant l'analogue de GLP-2 ;
dans lequel le quatrième système tampon inclut de l'acide acétique/acétate d'ammonium.

4. Procédé selon l'une quelconque des revendications précédentes, comprenant en outre le procédé de synthèse de l'analogue de GLP-2 par synthèse peptidique en phase solide (SPP), dans lequel Z² est une séquence peptidique de 1 à 6 unités d'acides aminés de Lys et dans lequel au moins une des unités de Lys dans Z² est protégée par un groupe protecteur de trityle pendant la synthèse.

5. Procédé selon la revendication 4, dans lequel Z² est une séquence peptidique de 2 à 6 unités d'acides aminés de Lys et le procédé comprend les étapes consistant à :
i) fixer un premier P-Lys (Trt)-OH à une résine peptidique à l'état solide à l'aide d'un lieur ;
ii) enlever le groupe P de l'unité d'acide aminé Lys (Trt) ;
iii) fixer un second P-Lys (Trt)-OH à l'unité d'acide aminé Lys(Trt) fixée à la résine peptidique à l'état solide à l'aide d'un lieur ;
iv) éliminer le groupe P à partir de la seconde unité d'acide aminé Lys(Trt) ;
v) fixer les unités d'acides aminés suivantes jusqu'à ce que l'analogue de GLP-2 soit synthétisé ;
vi) cliver l'analogue de GLP-2 à partir de la résine peptidique à l'état solide ;
vii) purifier l'analogue de GLP-2,
chaque P étant un groupe protecteur.

6. Procédé selon l'une quelconque des revendications précédentes, dans lequel X5 est Thr et/ou X11 est Ala.

7. Procédé selon la revendication 6, dans lequel l'analogue de GLP-2 est choisi parmi :
ZP1848 H-HGEGTFSSELATILDALAARDFIAWLIATKITDKKKKKK-NH₂ (SEQ ID NO : 1)
ZP2949 H-HGEGTFSSELATILDALAARDFIAWLIATKITDKKK-OH (SEQ ID NO : 2) ;
ZP2711 H-HGEGTFSSELATILDALAARDFIAWLIATKITDKK-OH (SEQ ID NO : 3) ;
ZP2469 H-HGEGTFSSELATILDALAARDFIAWLIATKITDK-OH (SEQ ID NO : 4) ;
ZP1857 H-HGEGTFSSELATILDALAARDFIAWLIATKITD-NH₂ (SEQ ID NO: 5) ; et
ZP2530 H-HGEGTFSSELATILDALAARDFIAWLIATKITD-OH (SEQ ID NO : 6).

8. Procédé selon la revendication 6, dans lequel l'analogue de GLP-2 est choisi parmi :
ZP1848 H-HGEGTFSSELATILDALAARDFIAWLIATKITDKKKKKK-NH₂ (SEQ ID NO : 1)
ZP2949 H-HGEGTFSSELATILDALAARDFIAWLIATKITDKKK-OH (SEQ ID NO : 2) ;
ZP2711 H-HGEGTFSSELATILDALAARDFIAWLIATKITDKK-OH (SEQ ID NO : 3) ; et
ZP2469 H-HGEGTFSSELATILDALAARDFIAWLIATKITDK-OH (SEQ ID NO : 4).

9. Procédé selon la revendication 6, dans lequel l'analogue de GLP-2 est :
ZP1848 H-HGEGTFSSELATILDALAARDFIAWLIATKITDKKKKKK-NH₂ (SEQ ID NO : 1).

10. Procédé selon l'une quelconque des revendications 1 à 5, dans lequel X5 est Ser et/ou X11 est Ser.

11. Procédé selon la revendication 10, dans lequel l'analogue de GLP-2 est choisi parmi :
ZP1846 H-HGEGSFSSELSTILDALAARDFIAWLIATKITDKKKKK-NH₂ (SEQ ID NO : 7) ;
ZP1855 H-HGEGSFSSELSTILDALAARDFIAWLIATKITD-NH₂ (SEQ ID NO : 8) ; et
ZP2242 H-HGEGSFSSELSTILDALAARDFIAWLIATKITDK-OH (SEQ ID NO : 9).

12. Procédé selon la revendication 10, dans lequel l'analogue de GLP-2 est :
ZP1846 H-HGEGSFSSELSTILDALAARDFIAWLIATKITDKKKKK-NH₂ (SEQ ID NO : 7).
